# EUROPEAN PATENT SPECIFICATION

(11) **EP 0 638 121 B1**
(45) Date of publication and mention of the grant of the patent: **17.07.1996**
(21) Application number: 93910848.6
(22) Date of filing: 28.04.1993
(51) Int. Cl.: C12N 15/11, A61K 31/70, C12N 9/00, C12Q 1/68, A61K 48/00

(54) **TARGETED CLEAVAGE OF RNA USING EUKARYOTIC RIBONUCLEASE P AND EXTERNAL GUIDE SEQUENCE**
GEZIELTE SPALTUNG VON RNA MITTELS EUKARYONTISCHER RNASE P UND EXTERNE FÜHRUNGSSEQUENZ
CLIVAGE CIBLE D'ARN AU MOYEN DE RIBONUCLEASE EUCARYOTE P ET D'UNE SEQUENCE-GUIDE EXTERNE

(30) Priority: 28.04.1992 US 875099; 18.08.1992 US 931937
(43) Date of publication of application: 15.02.1995
(73) Proprietor: YALE UNIVERSITY, New Haven, CT 06511 (US)
(72) Inventor: YUAN, Yan, New Haven, CT 06511 (US); GUERRIER-TAKADA, Cecilia, L., New Haven, CT 06515 (US); ALTMAN, Sidney, Hamden, CT 06517 (US)
(74) Representative: Bassett, Richard Simon
(86) International application number: US9303961
(87) International publication number: WO9322434

(56) References cited:
- WO-A-92/03566
- SCIENCE, vol. 249, 17 August 1990, Lancaster, PA (US); A. FORSTER et al., pp. 783-786
- PROCEEDINGS OF THE NATL. ACADEMY OF SCIENCES USA, vol. 89, 15 April 1992, Washington, DC (US); Y. LI et al., pp. 3185-3189
- PROCEEDINGS OF THE NATL. ACADEMY OF SCIENCES USA, vol. 89, September 1992, Washigton, DC (US); Y. YUAN et al., pp. 8006-8010

## Description

### Background of the Invention

This invention is in the general area of genetic engineering of nucleic acid sequences, especially RNA sequences that are substrates for Ribonuclease P.

This is a continuation-in-part of U.S. Serial No. 07/931,937 filed August 18, 1992 entitled "Targeted Cleavage of RNA Using Eukaryotic Ribonuclease P and External Guide Sequence", by Yan Yuan, Cecilia Guerrier-Takada and Sidney Altman, which is a continuation in part of 07/875,099 filed April 28, 1992; which is a continuation in part of U.S. Serial No. 07/568,834 entitled "Therapeutic Ribozyme Compositions" filed August 1990 by Sidney Altman, et al., issued as U.S. Patent No. 5,168,053, which is a continuation in part of U.S. Serial No. 07/328,368 entitled "Removing or Inactivating Undesired RNA" filed March 24, 1989 by Sidney Altman, Anthony C. Forster, and Cecilia L. Guerrier-Takada, now abandoned.

The United States government may have certain rights in this invention as a result of grants from the National Institutes of Health and National Science Foundation, GM 19422 and DMB 9101670 to Sidney Altman.

Discoveries in the realm of molecular biology over the past five years have led to the realization that RNA has a series of distinct capabilities and biological activities previously unsuspected. The most important of these novel RNA-level discoveries has been the finding that RNA can be an enzyme as well as an information carrier.

There are several classes of ribozymes now known which are involved in the cleavage and/or ligation of RNA chains. A ribozyme is defined as an enzyme which is made of RNA, most of which work on RNA substrates. Ribozymes have been known since 1982, when Cech and colleagues (Cell, 31: 147-157) showed that a ribosomal RNA precursor in *Tetrahymena,* a unicellular eukaryote, undergoes cleavage catalyzed by elements in the RNA sequence to be removed during the conversion of the rRNA precursor into mature rRNA. This sequence to be removed (called an intervening sequence or intron) is one of what are now known to be numerous examples of "Class I" intron ribozyme activities. A similar "Class II" intron ribozyme mechanism was discovered more recently, involving the cleavage and subsequent ligation of a number of yeast mitochondrial RNAs (Nature, 324: 429-433 1987). Cech and colleagues described certain *in vitro* applications of "class I" ribozymes in PCT/US887/03161 by University Patents, Inc., (published as WO 88/04300 16 June 1988). Their potential for therapeutic applications in cells and in patients remains unclear.

A third class of ribozyme, discovered in 1983, was the first to be shown to work in trans (i.e., to work under conditions where the ribozyme is built into one RNA chain while the substrate to be cleaved is a second, separate RNA chain). This ribozyme, called M1 RNA, was characterized in 1983 by Altman and colleagues as responsible for the cleavage which forms mature 5' ends of all transfer RNAs (tRNAs) in E. coli. Analogous RNA-containing enzymes concerned with tRNA synthesis have since been found in all cells in which they have been sought, including a number of human cell lines, though the relevant eucaryotic RNAs have not yet been shown to be catalytic by themselves *in vitro.*

The discovery and characterization of this catalytic RNA is reviewed by Sidney Altman, in "Ribonuclease P: An Enzyme with a Catalytic RNA Subunit" in Adv. Enzymol. 62, 1-36 (1989). The activity was first isolated from *E. coli* extracts, and subsequently determined to be a ribonucleoprotein having two components, an RNA component called M1 and a protein component called C5. The RNA cleaved substrates in a true enzymatic reaction, as measured using Michaelis-Menton kinetics. M1 was determined to be solely responsible for substrate recognition and C5 was determined to alter k_{cat} but not K_{M}, as reported by Guerrier-Takada, et al., Cell 35, 849 (1983) and McClain, et al., Science 238, 527 (1987). Sequencing showed that M1 RNA is 377 nucleotides long, Mᵣ approximately 125,000, and that the protein consists of 119 amino acids, Mᵣ approximately 13,800, as reported by Hansen, et al., Gene 38, 535 (1987).

Two other ribozyme classes are related to the replication cycle of a group of self-replicating RNAs called "viroid-like pathogens", or VLPs. Plant viroids, RNA satellites of plant viruses, and Hepatitis delta virus are all members of the VLP group. The VLPs can be divided into two classes: Class I, free living viroids; and Class II, including virusoids and satellite viroids (RNA molecules which require a helper virus to replicate). The hepatitis delta virus is a Class II VLP by this definition.

In 1984, Branch and Robertson (Science, 233: 450-455) published the replication cycle strategies for these pathogens, subsequently verified by experiments conducted in several laboratories. A key element of this "rolling-circle" replication strategy is that the VLP undergoing replication makes greater-than-unit-length copies of its information, which are then cleaved to monomeric size by ribozyme activities built into the RNA of the VLP itself. Sharmeen at. al., J. Virol., 62, 2674-2679 (1988); Branch, et. al., Science, 243, 649-652 (1989); and Wu and Lai, Science 243, 652-655 (1989), defined the ribozyme cleavage points of both delta strands and the domains containing them for hepatitis delta virus.

One type of VLP ribozymes is defined by a small structural domain, consisting of only about 30 nucleotides, called a "hammerhead". Uhlenbeck, Nature (1987), first developed these small (as few as 18 nucleotides) and relatively specific ribozyme sequences from plant viroids such as avocado sunblotch viroid and the satellite RNAs of tobacco ringspot virus and lucerne transient streak virus. Uhlenbeck (1987) and Forster and Symons (Cell 50, 9-16, 1987), defined the requirements for cleavage by this ribozyme class. Various embodiments and potential applications have also been described by Haseloff, Gerlach and Jennings in PCT/AU88/00478 by Commonwealth Scientific and Industrial Research Organization (published as WO 90/05852 29 June 1989).

All reactions that are governed by RNA *in vivo* result in the transesterification or hydrolysis of specific phosphodiester bonds in RNA. In several classes of these reactions, an intramolecular site of cleavage or ligation is identified by internal guide sequences (IGSs) which form base pairs with the segment of the phosphodiester chain that contains the cleavage site. The *Tetrahymena* sequence, as well as the subsequently discovered sequence in yeast, is not a true enzyme since it is not regenerated in the process but instead acts in a stoichiometric ratio. Although it is possible to engineer fragments of this sequence which have enzymatic activity under certain conditions *in vitro* and are able to cleave and ligate RNA, a disadvantage to these fragments is that they are very large (requiring more than 200 residues of the original 415 nucleotide sequence) and of limited specificity. In their present forms, the *Tetrahymena* ribozymes have four-base recognition sequences and the hammerhead ribozymes have approximately 12-base recognition sequences, allowing these ribozymes to be used in a complementary fashion to cleave RNA.

IGSs are not present in one class of reactions governed by RNA that is enzymatic *in vivo,* cleavage of precursor tRNA molecules by the RNA component of eubacterial RNAase P, described by Guerrier-Takada, et al., Cell 35, 849 (1983) and reviewed by Altman, Adv. Enzymol. 62, 1 (1989). The nucleotide sequence of the segment of the phosphodiester chain that contains the cleavage site is not conserved among different substrates for RNAase P, so it cannot be recognized as a unique IGS for the enzyme.

U.S. Serial No. 07/568,834 entitled "Therapeutic Ribozyme Compositions" filed August 1990 by Sidney Altman, et al., and U.S. Serial No. 07/328,368 entitled "Removing or Inactivating Undesired RNA" filed March 24, 1989 by Sidney Altman, et al., disclose that it is possible to target any RNA molecule for cleavage by bacterial RNAase P by forming a nucleotide sequence part of which is complementary to a targeted site and which includes a terminal 3'-NCCA, wherein the sequence is designed to hybridize to the targeted RNA so that the bacterial RNAase P cleaves the substrate at the hybrid base-paired region. Specificity is determined by the complementary sequence. The sequence is preferably ten to fifteen nucleotides in length and may contain non-complementary nucleotides to the extent this does not interfere with formation of several base pairs by the complementary sequence which is followed by NCCA at the 3' end.

Subsequent studies have demonstrated that the external guide sequence, or "EGS", that is useful in targeting procaryotic RNAase P, does not result in cleavage of a targeted RNA strand by eukaryotic RNAase P.

It is therefore an object of the present invention to provide methods and compositions for specifically cleaving targeted RNA sequences using eukaryotic RNAase P or functional equivalents thereof.

It is a further object of the present invention to provide methods and compositions for specifically cleaving RNA, both *in vitro* and *in vivo* within eukaryotic cells, for the treatment of disease conditions which involve RNA transcription or translation, such as diseases caused by RNA and DNA viruses and expression of excessive or pathogenic proteins from mRNA, or of excessive or pathogenic RNA, itself.

### Summary of the Invention

It has been discovered that any RNA can be targeted for cleavage by RNAase P from human cells, using a suitably designed oligoribonucleotide ("external guide sequence", or EGS) to form a hybrid with the target RNA, thereby creating a substrate for cleavage by RNAase P *in vitro.* The data indicate that it is reasonable to conclude that these observations can be extended to RNAase P from any eucaryotic cell. There are two classes of EGS that can target RNA for cleavage by human RNAase P. These are prepared by transcription from a small DNA fragment that contains a sequence coding for an RNA that can assume a tRNA-like secondary structure. In the first class, the structure lacks at least the first thirteen nucleotides from the 5' terminus of the tRNA-like sequence, the anticodon stem and loop, or the variable loop or part of the variable loop. The most efficient EGS of this first class with human RNAase P is the EGS in which the anticodon stem and loop was deleted. In the second class, the EGS has changes in the equivalent of the T-loop, the variable loop, and the anticodon stem of the tRNA-like segment of the EGS. Methods are also disclosed to select a suitable EGS of the second class for use *in vivo* to make a selected RNA a target for cleavage by the host cell RNAase P, thus preventing expression of the function of the target RNA. The methods and compositions should be useful to prevent the expression of disease-causing genes *in vivo.*

### Brief Description of the Drawings

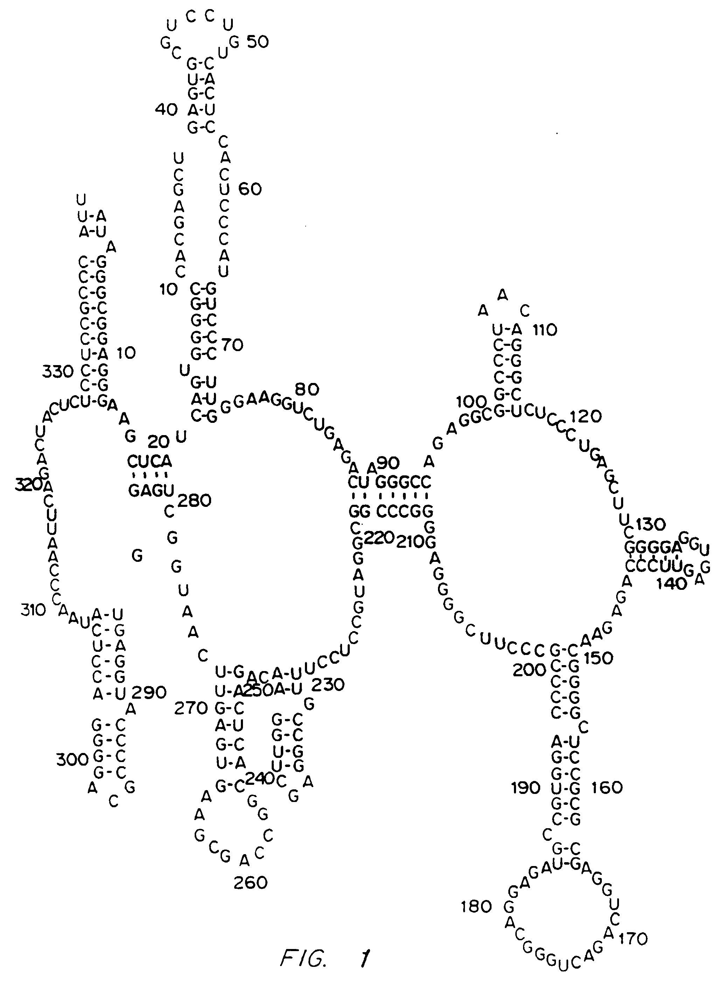

Figure 1 is the sequence and structure of H1 RNA.

Figure 2 is a general scheme of an EGS for use in targeting eukaryotic RNAase P to cleave a specific substrate, with the arms and stems characteristic of a tRNA labeled.

Figure 3a is the sequence and secondary structures of the precursor to tRNA^{Tyr} and a complex of a substrate with an EGS derived tRNA^{Tyr}. pTyr: *E. coli* tRNA^{Tyr} precursor; pAva: substrate (target) RNA with 5' leader sequence and the first fourteen nucleotides of *E. coli* tRNA^{Tyr}. Figure 3b is an autoradiograph of a gel electrophoresis analysis of the cleavage reaction. Arrows mark the expected cleavage sites.

Figure 4 is a schematic representation of complexes of chloramphenicol acetyltransferase mRNA and the EGS^{CAT} RNAs.

Figures 5a and 5b are schematics of one class of EGS for use in targeting eukaryotic RNAase P to cleave a specific substrate: Figure 5a is an EGS corrresponding to the EGS in Figure 2; Figure 5b is an EGS where the anticodon stem and loop have been removed.

Figures 6a, 6b, and 6c are schematics of a second class of EGS for use in targeting eukaryotic RNAase P to cleave a specific substrate where the extra loop, the T loop, and the anticodon stem have been modified.

### Detailed Description of the Invention

Ribonuclease P from *E. coli* can cleave oligoribonucleotides that are found in hydrogen-bonded complexes that resemble the aminoacyl stem and 5' leader sequence of tRNA precursors. RNAase P from human (HeLa) cells cannot cleave *in vitro* the 5' proximal oligoribonucleotide in such simple complexes but can do so when the 3' proximal oligoribonucleotide (external guide sequence or EGS) resembles portions of a tRNA molecule.

Examples provided below show the efficiency of cleavage of the mRNA for chloramphenicol acetyltransferase (CAT) by human RNAase P. In such a complex, the sequence of the 5' oligoribonucleotide, as well as that of the EGS, depend on the choice of target site in the mRNA. The presence of the appropriately designed EGS efficiently reduces CAT enzymatic activity *in vivo,* as well as promotes cleavage of CAT mRNA *in vitro,* indicating that this method should be of general use for gene inactivation.

### Ribonuclease P.

Ribonuclease P is an enzyme consisting of protein and RNA subunits that cleaves tRNA precursors to generate the 5' termini of tRNAs. This essential enzymatic activity has been found in all cell types examined, both prokaryotic and eukaryotic. During the studies on recognition of the substrate by RNAase P, it was found that *E. coli* RNAase P can cleave synthetic tRNA-related substrates that lack specific domains (the D, T and anticodon stems and loops) of the normal tRNA structure. A half-turn of an RNA helix and a 3' proximal CCA sequence contain sufficient recognition elements to allow the reaction to proceed. The 5' proximal sequence of the RNA helix does not have to be covalently linked to 3' proximal sequence of the helix. The 3' proximal sequence of the stem can be regarded as an "external guide sequence" (EGS) because it identifies the site of cleavage in the 5' proximal region through a base-paired region.

Theoretically, any RNA of known sequence could be targeted by a custom-designed EGS RNA for specific cleavage by RNAase P. EGS-directed mRNA cleavage has been achieved with *E. coli* RNAase P *in vitro.*

RNAase P from *E. coli* and human cells have similar but not identical biochemical properties. Their RNA components have similar secondary structures. However, the substrate range of human RNAase P is much narrower than that of the *E. coli* enzyme. For example, although *E. coli* RNAase P can cleave a synthetic tRNA-related substrate that lacks three specific domains (the D, variable, and anticodon stem and loop) of the normal tRNA structure, it is not a substrate for the human enzyme or that from the yeast, *S. cerevisiae.* Therefore, the simplest design of an EGS for RNA cleavage by RNAase P from human and presumably other eukaryotic cells has to be different from that for cleavage by *E. coli* RNAase P.

As used herein, unless otherwise specified, RNAase P refers to the RNAase P in the cell in which the RNA to be cleaved is located, whether endogenous, added to the cell, or as used *in vitro.* Many of the techniques described herein are known to those skilled in the art, as are methods for making, and sources of, reagents. The teachings of any references cited herein with respect to methods and reagents are specifically incorporated herein, as well as for the purpose of demonstrating the scope and level of skill in the art.

### The External Guide Sequence

### A. RNAase P targeting Sequence.

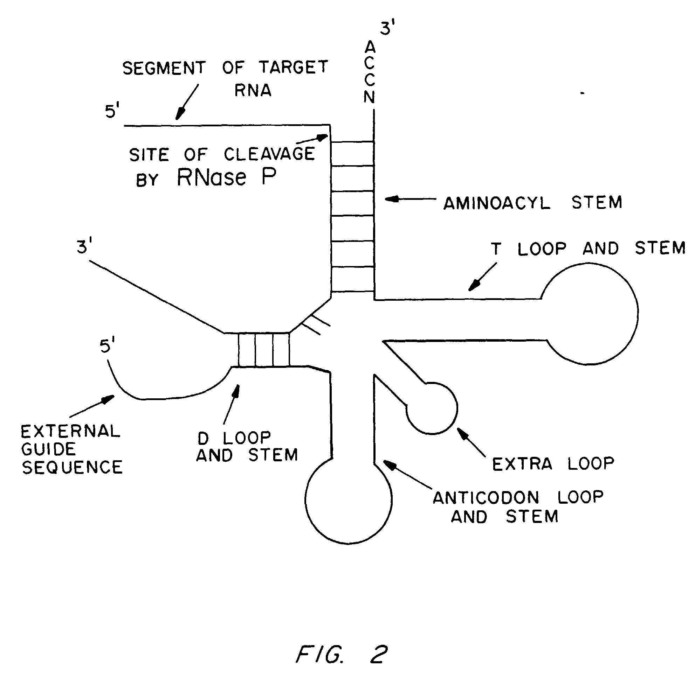

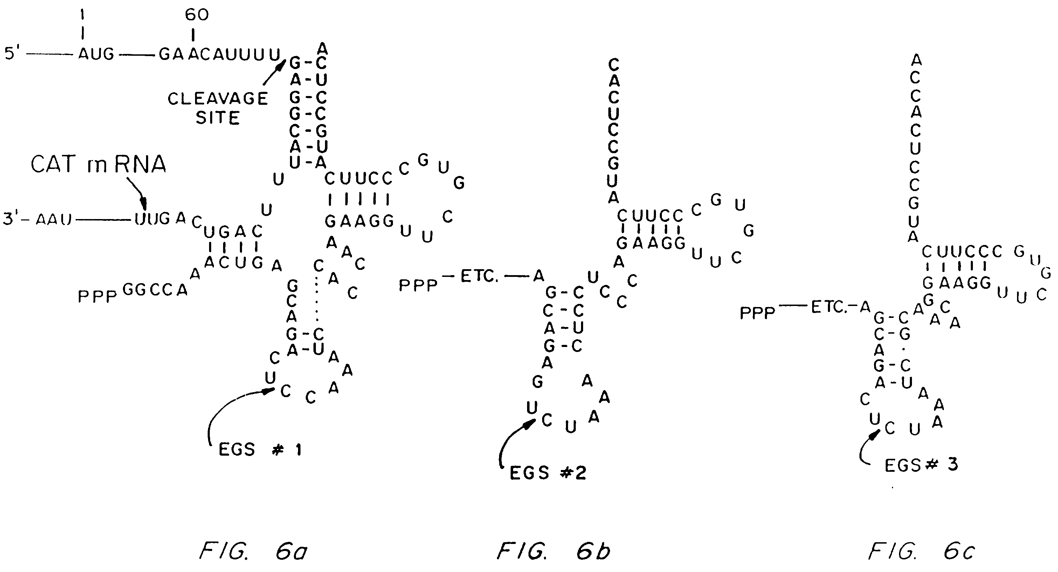

As shown by Figures 2, 3, 4, 5 and 6, an EGS for a human RNAase P consists of a sequence which, when in a complex with the target substrate molecule, forms a secondary structure resembling that of a tRNA cloverleaf or a substantial part of it. As used herein, the term "resembling a precursor tRNA" means a sufficient portion of the tRNA secondary structure to form a complex which will result in cleavage of the target RNA by RNAase P. The sequence of the EGS can be derived from any tRNA except that the D stem and aminoacyl stem have to be altered to be complementary to the target substrate sequence and the apparent D stem contains four base pairs. The presence of the 3'-CCA enhances the efficiency of the reaction with the human RNAase P by about 35%. The anticodon loop and stem and extra loop can separately be deleted and the T loop and stem can be modified without decreasing the usefulness of the EGS and, in the case of the anticodon stem and loop deletion, increases the efficiency of the reaction by about ten fold. Changes in other parts of an EGS (see Figure 6) can increase its efficiency about one hundred fold.

The desired secondary structure is determined using conventional Watson-Crick base pairing schemes to form a structure resembling a tRNA, i.e., having structure as described below. The specific sequence of the hydrogen bonded regions is not so critical, as long as the desired structure is formed. All tRNAs, including tRNAs from a wide variety of bacteria and eukaryotes, conform to the same general secondary structure. This is typically written in the form of a cloverleaf, maintained by hydrogen-bonded base pairing between short complementary regions. The four major arms are named for their structure or function: The acceptor arm consists of a 3' terminal CCA_{OH} plus a variable fourth nucleotide extending beyond the stem formed by base-pairing the 5' and 3' segments of the molecule. The other arms consist of base-paired stems and unpaired loops. The "T" arm is named for the presence of the ribothymidine nucleotide and contains seven unpaired bases. The anticodon arm always contains the anticodon triplet in the center of the loop and consists of seven unpaired bases. The D arm is named for the presence of the base dihydrouridine, another of the modified bases in tRNA, and includes between eight and twelve unpaired bases. Positions are numbered from 5' to 3' according to the most common tRNA structure, which has 76 residues. The overall range of tRNA lengths is from 74 to 95 bases. The variation in length is caused by differences in the structure of two of the arms, the D arm and the extra arm, which lies between the T and anticodon arms, which can contain between three and five bases, or between 13 and 21 bases with a stem of about five bases. The base pairing that maintains the secondary structure is virtually invariant: there are always seven base pairs in the acceptor stem, five in the T arm, five in the anticodon arm, and three or four in the D arm.

As used herein, a hybrid structure having secondary structure resembling a precursor tRNA under conditions promoting cleavage by the RNAase P of the substrate at the nucleotide at the 5' end of the base-paired region, preferably includes a D loop and stem, an aminoacyl stem, an anticodon loop and stem, a variable loop and stem, and a T loop and stem, where the latter three may be modified compared to the sequence and detailed structure found in the parent molecule.

A few nucleotides are always found in the same positions in 90 to 95% of tRNAs, with some additional nucleotides being semiconserved (or semivariant). This is not a requirement in the EGS, as long as the sequence is complementary to the target and forms the secondary structure characteristic of the tRNA. As shown in Figure 2, the sequence forming the aminoacyl stem and D loop and stem are changed in the EGS to be complementary to the target RNA.

The base paired double-helical stems of the secondary structure are maintained in the tertiary structure, creating two double helices at right angles to each other. The acceptor stem and the T stem form one continuous double helix with a single gap; the D stem and the anticodon stem form another continuous double helix, also with a gap. Many of the invariant and semi-invariant bases are involved in the tertiary structure.

### B. Complementary Sequence.

The complementary sequences will generally consist of eleven nucleotides (see Figure 2) in two blocks as shown, separated by two unpaired nucleotides in the target sequence, preferably UU, wherein the two blocks are complementary to a sequence 3' to the site targeted for cleavage.

### Ribozyme Activity

It is not necessary to provide ribozyme activity if the cleavage is to occur intracellularly in the nucleus since all cells contain RNAase P in their nuclei. RNAase P must be supplied if cleavage is to occur in the cytoplasm. As used herein for ease of convenience, RNAase P refers to the ribonucleoprotein consisting of the eukaryotic analogues of the *E. coli* C5 protein and M1 RNA, regardless of source, whether isolated, produced by chemical synthesis or, in the case of the RNA, transcription from the gene, referred to herein as H1 RNA. The RNA subunit need not necessarily manifest catalytic activity in the absence of protein subunits *in vitro.*

### A. Endogenous RNAase P.

The sequence and proposed secondary structure of H1 RNA, the RNA component of human RNAase P, was reported by Baer, et al., in Nucleic Acids Res. 18(1), 97-103 (1989), the teachings of which are incorporated herein. The sequence of H1 DNA (**Sequence ID No. 1**) is as follows:

ATGGGCGGAG GGAAGCTCAT CAGTGGGGCC ACGAGCTGAG TGCGTCCTGT
CACTCCACTCCCATGTCCCT TGGGAAGGTC TGAGACTAGG GCCAGAGGCG
GCCCTAACAG GGCTCTCCCTGAGCTTCAGG GAGGTGAGTT CCCAGAGAAC
GGGGCTCCGC GCGAGGTCAG ACTGGGCAGGAGATGCCGTG GACCCCGCCC
TTCGGGGAGG GGCCCGGCGG ATGCCTCCTT TGCCGGAGCTTGGAACAGAC
TCACGGCCAG CGAAGTGAGT TCAATGGCTG AGGTGAGGTA CCCCGCAGGG
GACCTCATAA CCCAATTCAG ACCACTCTCC TCCGCCCATT.

The nucleotide sequence of H1 DNA was determined from the cDNA sequence. The underlined part of the sequence was determined by RNA sequence analysis.

Because of the similarity in secondary structure and substrate specificity among the RNAase P's of diverse origin, it is possible to use an EGS designed to maximize efficiency of cleavage for the RNAase P in question using techniques described herein to target any RNA in any cell, even though the catalytically active RNA subunits may have distinctly different sequences. See Altman, Ann. Rev. Enzymology 62, 1-39(1989); Altman, J. Biol. Chem. 265, 20053-20056 (1990). Secondary structure is defined by intramolecular associations of complementary sequences. Base pairs can be canonical, A/U and G/C, or non-canonical, G/U, A/G, etc.

### B. Exogenous RNA having catalytic activity.

An EGS can also be used in combination with an RNA sequence that demonstrates enzymatic activity in the presence or absence of a protein. That RNA sequence can be represented by a molecule like the entire H1 RNA molecule or any portion thereof shown to have catalytic activity in combination with a protein, or any functionally equivalent molecule of eukaryotic origin or derivation.

There are two principle situations in which catalytic exogenous RNA or RNAase P is utilized in combination with EGS: *in vitro* in the absence of cells or cellular RNAase P and in circumstances wherein the RNA to be cleaved is located in a portion of a cell not containing endogenous RNAase P. In the latter case, the genes encoding the analogs of M1 RNA and C5 protein (as defined above), or the human or other eucaryotic equivalents thereof, are introduced into the cell at the desired location for cleavage using a suitable vector or other method known to those skilled in the art for introduction and expression of a gene in a cell.

### Application of the EGS as laboratory or clinical reagents.

The external guide sequences have applications as *in vitro* reagents, in a similar fashion to restriction enzymes, and as therapeutic agents, for cleavage and inactivation of specific host cell RNA or RNA coded for by pathogenic organisms such as bacteria or viruses. The external guide sequence, consisting of the combination of eleven bases complementary to a sequence on the 3' side of the desired cleavage site and specific to the targeted RNA and a 3' terminal NCCA sequence, can be added to any RNA having sequences complementary to the EGS, in the presence of RNAase P, and the RNA will be cleaved at the targeted site. In this manner, the activity of endogenous RNAase P in any cell, such as the RNAase P of human cells, can be directed to destroy specific messenger, viral or other RNAs by the use of an appropriate EGS RNA.

### 1. Reagents for in vitro applications.

DNA restriction endonucleases are invaluable reagents for the molecular biologist. Patterns of restriction fragment sizes are used to establish sequence relationships between DNA molecules, and large DNAs can be cleaved to give fragments of sizes useful for genetic engineering, sequencing, and studying protein binding. RNA processing enzymes can be utilized under conditions such that they also cleave RNA with considerably sequence specificity.

Specific ribozymes can be prepared by combining the specific guide sequence with eukaryotic RNAase P or functional equivalents thereof. In the preferred embodiment, the external guide sequence and the RNA subunit of RNAase P (e.g., H1 RNA) are separate; alternatively, the two sequences can be combined using an oligonucleotide linker that allows sufficient flexibility between the targeting sequence and the H1 RNA (or equivalent) sequence for the targeting guide sequence to bind and the catalytic sequence, whether in association with an analog of C5 protein or not, to cleave.

### 2. Therapeutics.

### a. Determination and Preparation of Complementary Sequences.

Any cellular gene product expressed as RNA, including proteins encoded by mRNA and structural RNAs themselves, can be targeted for inactivation by RNAase P using sequences engineered to include appropriate regions of sequence and/or structure for binding to the targeted RNA and the desired site of cleavage. The cellular gene product could be a modified product of an oncogene, such as the ras gene product; where the product is not a normal cell component, a viral protein, such as one encoded by an essential gene for HIV replication; or a bacterial protein.

In many cases, the critical genes in an infective or pathological agent have been isolated and sequenced. Appropriate complementary sequences can be synthesized using standard techniques, reagents, and equipment based on these known sequences.

### b. Preparation of an appropriate pharmaceutical composition for delivery of the EGS to the targeted RNA.

There are two primary mechanisms for delivering the EGS to intracellular RNA that has been targeted for cleavage: diffusion and via a vector.

As discussed above, any RNA that is important in a disease process can be targeted and appropriate complementary sequences made synthetically or by copying cloned sequence. Since RNAase P is predominantly found in the nucleus of eukaryotic cells, the infectious agents most likely to be inhibited by administration of appropriate EGS to the infected cells are those in which critical RNA sequences are transcribed in the nucleus. Important examples of the viral agents that replicate in the cell nucleus include herpesviruses (including herpes simplex virus, varicella-herpes zoster virus, cytomegalovirus, and Epstein-Barr virus), hepatitis B virus, adenoviruses, paramyxoviruses such as measles, and the retroviruses, such as human immunodeficiency virus (HIV I, HIV II, HIV III and HTLV-1). **Vector-mediated delivery of EGS.**

Preferred vectors are viral vectors such as the retroviruses which introduce the EGS directly into the nucleus where it is transcribed and released into the nucleus. Under the appropriate conditions, the EGS will hybridize to the targeted RNA and the endogenous RNAase P will cleave the hybridized RNA at the 5' side of the hybrid region.

Methods for using retroviral vectors for gene therapy are described in U.S. Patent Nos. 4,868,116 and 4,980,286, and PCT application PCT/US89/03794 and PCT/US89/00422, the teachings of which are incorporated herein.

Defective retroviral vectors, which incorporate their own RNA sequence in the form of DNA into the host chromosome, can be engineered to incorporate the EGS into the host, where copies will be made and released into the cytoplasm to interact with the target nucleotide sequences.

The ability to introduce specifically engineered nucleic acid sequences, as a means of targeted therapy, into hematopoietic cells of patients suffering from virus-induced disease of those cells, such as AIDS, has great potential. The most efficacious methodology presently available for the introduction of specific genetic sequences into human cells involves the use of RNA-containing retroviruses which serve as vehicles or vectors for high efficiency gene transfer into human cells.

RNAase P-based therapy can also be used as a means of preventing the spread of HIV-1 and or providing a HIV-1 resistant population of T-cells that will be able to confer immune function to infected individuals. Patients who have been recently diagnosed as having antibodies to HIV-1, but who do not yet show AIDS symptomatology, are the most likely be the best candidates for therapy. This procedure will necessitate removal of some of the patient's bone marrow stem cells and subsequent partial cytoblation. The removed cells can be treated in the laboratory with appropriate EGS compositions (via appropriate viral vectors, such as defective viral vectors) and then restored to the same individual. The treated cells will develop in the patient into mature hematopoietic cells, including T-cells. These T-cells will have normal immune function and, most importantly, will be intracellularly immunized to prevent their destruction by any HIV-1 still present in the patient.

Bone marrow stem cells and hematopoietic cells are relatively easily removed and replaced and provide a self-regenerating population of cells for the propagation of transferred genes. As described above, HIV-1 and HTLV-1 should be amenable to these approaches. In the longer term, it is anticipated that the use of RNAase P-based therapeutics will allow the selective inactivation of other unwanted genes in cells, such as activated oncogenes, involved in the causation and maintenance of cancer cells.

In contrast to the approaches presently in use which are aimed at preventing or limiting infection with HIV, it should be possible to use RNAase P-based technology to treat, and possibly to cure, HIV infection, and related diseases of white blood cells which are subject to transformation by retroviral vectors carrying EGS. Particular examples of diseases that may be treated using EGS to target RNA for cleavage by RNAase P include not only HTLV-1, but also various retroviral-induced leukemias resulting from chromosomal translocations that produce chimeric RNAs which produce proteins that are unique to those cells and that can act as growth stimulators or oncogenes. Other types of transformed tissues that might be treatable include all cancer cells carrying identified oncogenes of known sequence.

### Topical and other EGS compositions for direct administration.

The EGS may also be administered topically or systemically in a suitable pharmaceutical carrier. Remington's Pharmaceutical Sciences, 15th Edition by E.W. Martin (Mark Publishing Company, 1975), the teachings of which are incorporated herein by reference, discloses typical carriers and methods of preparation. The EGS may also be encapsulated in suitable biocompatible microcapsules or liposomes for targeting to phagocytic cells. Such systems are well known to those skilled in the art.

Therapeutically the oligoribonucleotides are administered as a pharmaceutical composition consisting of an effective amount of the EGS to inhibit transcription of a targeted RNA and a pharmaceutically acceptable carrier. Examples of typical pharmaceutical carriers, used alone or in combination, include one or more solid, semi-solid, or liquid diluents, fillers and formulation adjuvants which are non-toxic, inert and pharmaceutically acceptable. Such pharmaceutical compositions are preferable in dosage unit form, i.e., physically discreet units containing a predetermined amount of the drug corresponding to a fraction or multiple of the dose which is calculated to produce the desired therapeutic response, conventionally prepared as tablets, lozenges, capsules, powders, aqueous or oily suspensions, syrups, elixirs, and aqueous solutions. It is essential that the oligonucleotides be delivered in a form which prevents degradation of the oligonucleotide before it reaches the intended target site. Examples of preferred means for delivery are polymeric matrices such as the biodegradable polymers polylactic acid and Pluronics.

A preferred composition is a topical composition, for example, for application to a viral lesion such as that produced by herpes simplex virus. These will generally contain between 1 µM and 1 mM oligonucleotide/unit of carrier, or produce a concentration between 1 µM and 1 mM at the site of the cells to be treated. Oral compositions, although not preferred, are in the form of tablets or capsules and may contain conventional excipients such as binding agents, (e.g., syrup, acacia, gelatin, sorbitol, tragacanth or polyvinylpyrrolidone), fillers (e.g., lactose, sugar, corn starch, calcium phosphate, sorbitol, or glycine), lubricants (e.g., magnesium stearate, talc, polyethylene glycol or silica), disintegrants (e.g., starch) and wetting agents (e.g., sodium lauryl sulfate). Solutions or suspensions of the EGS with conventional pharmaceutical vehicles are employed for parenteral compositions, such as an aqueous solution for intravenous injection or an oily suspension for intramuscular injection.

For clinical applications, the dosage and the dosage regimen in each case should be carefully adjusted, utilizing sound professional judgment and consideration of the age, weight and condition of the recipient, the root of administration and the nature and gravity of the illness.

The present invention, a RNA sequence targeting a second RNA sequence for cleavage by eukaryotic RNAase P, will be further understood by reference to the following non-limiting examples.

### Example 1: Cleavage of tRNA precursor fragments by human RNAase P in the presence of EGS.

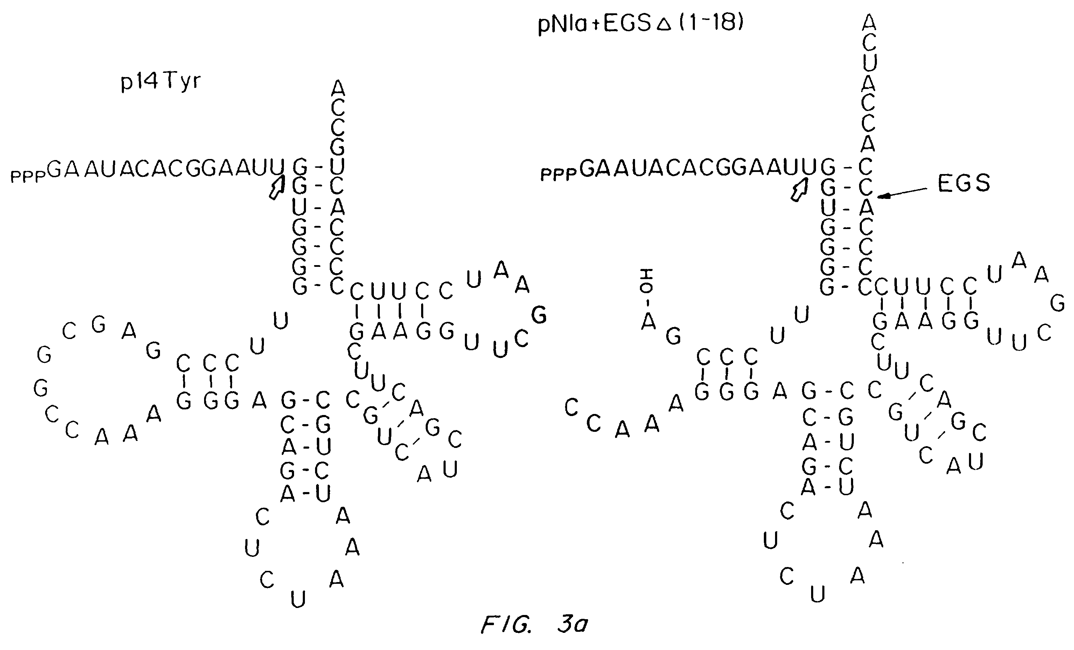

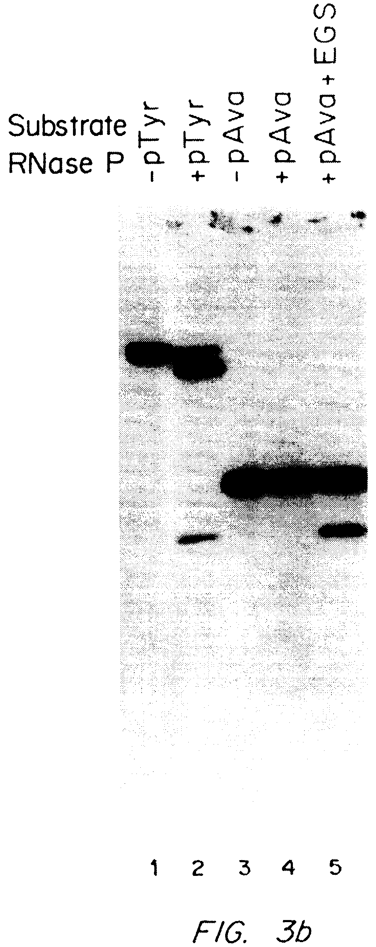

An EGS that can target RNA for cleavage by human RNAase P was prepared from a small RNA fragment which contains a 5' precursor sequence and the first fourteen nucleotides from the 5' terminus of a tRNA (Sequence ID No. 2, shown in Figure 3a). The leader sequence of *E. coli* tRNA^{Tyr} precursor can be cleaved correctly when another piece of RNA which contains the remaining 3' proximal sequence of tRNA^{Tyr} sequence is hybridized to the target, as shown in Figure 3a and b, demonstrating cleavage of a substrate by human RNAase P in the presence or absence of EGS RNAs.

Human RNAase P was partially purified from HeLa cells according to Bartkiewicz et al. (1989) Genes and Development 3:488-499. The substrates were prepared by *in vitro* transcription in the presence of [α-³²P]GTP. [α-³²P]GTP labelled pAva I RNA (28 nt) was mixed with unlabelled EGS RNA and the mixture was incubated at 37°C in 50 mM Tris-Cl (pH 7.5), 100 mM NH₄Cl, and 10 mM MgCl₂ with enzyme for 30 min. Labelled pAva I RNA alone was also incubated with or without enzyme. Analysis by gel electrophoresis shows that the EGS plus enzyme resulted in cleavage of the RNA.

The 3' fragment is the external guide sequence (EGS). Because the lengths of the leaders and their sequences, as well as the sequences of the mature domain, are not conserved among different precursor-tRNAs, the main determinants for human RNAase P cleavage must be in some of the conserved structural features of various tRNAs. This general idea is borne out by the fact that several other EGSs that did not mimic exactly the structure of parts of a tRNA (i.e., changes in the number of possible base pairs in the D or amino acyl stems, changes in positions 8 and 9 of the mature tRNA sequence, and a change from cytosine to uracil at position 57) did not target complementary RNAs. However, an EGS that lacked the anticodon stem and loop, or the variable stem and loop, resulted in efficient cleavage, indicating that these parts of the EGS, separately, were not essential for recognition of the target complex by the enzyme.

Presumably, if an mRNA, rather than part of a precursor tRNA sequence, is incorporated into the double-stranded stem region of a putative target complex, and the resulting hybrid contains the structural features required of a substrate for human RNAase P activity, the mRNA should be cleaved by human RNAase P.

### Example 2: Specific cleavage of CAT mRNA in vitro by human RNAase P using an external guide sequence.

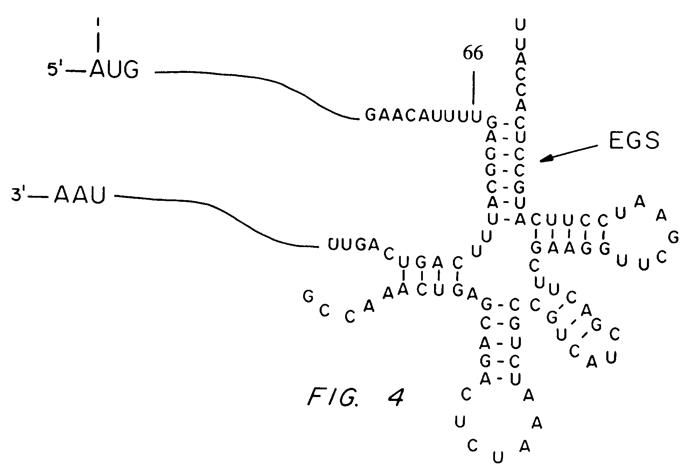

The mRNA for the gene for chloramphenicol acetyltransferase (CAT) can be easily manipulated on plasmids and the enzymatic activity is readily expressed in tissue culture cells so it was used as a target substrate. As demonstrated below, an EGS can target CAT mRNA for specific cleavage by human RNAase P. Figure 4 shows a complex in which an EGS (Sequence ID No. 3) could base-pair with nucleotides 67 to 79 of CAT mRNA and direct human RNAase P to cleave that mRNA at nucleotide 67 (The first nucleotide of translation initiation codon is numbered 1). The EGS^{CAT} construct was derived from the *E. coli* tRNA^{Tyr} gene where the first eighteen nucleotides from the 5' terminus have been deleted and the sequences on the D-loop and acceptor-loop have been changed to make base-pairs with CAT mRNA. The EGS^{CAT} fused upstream with a T7 promoter was cloned into a pUC19 vector. The EGS^{CAT} RNA was prepared through *in vitro* transcription with T7 RNA polymerase. A *Hind* III - *Bam* HI DNA fragment of the CAT gene (pCAT™, Promega) was cloned in pGem-2. The CAT DNA was linearized with *Eco*RI and transcribed with T7 RNA polymerase in the presence of [³²P-α]GTP. Labeled CAT mRNA was mixed with various amounts of the EGS^{CAT} molecule without special treatment of denaturing or annealing and susceptibility to cleavage by RNAase P *in vitro* was tested with human RNAase P partially purified from HeLa cells.

The *Hind* III -- *Bam* HI fragment of CAT gene (pCAT™, Promega) was cloned in pGem-2. The plasmid was truncated with *Eco*RI and a 260 nucleotide-long transcript was obtained by *in vitro* transcription with T7 RNA polymerase in the presence of [α-³²P]GTP. The EGS sequence was synthesized by polymerase chain reaction, using the *E. coli* tRNA^{Tyr} gene as template, with oligonucleotide 5'-GCCAAACTGAGCAGACTC-3' and 5'-GCGCggtaccAAAAATGGTGAGGCATGAAGG-3', where the bold letters in the oligonucleotide sequences indicate the bases needed to make base pairs to CAT mRNA, the underlined letters indicate the sequence complementary to the transcription termination signal, and the lower case letters shows an extra linker sequence (GCGC at the 5' end of the second oligonucleotide are extra nucleotides). The PCR fragment was digested with *Hind* III and cloned into pUC19 with a T7 promoter upstream from the EGS sequence. The EGS^{CAT} RNA was transcribed with T7 RNA polymerase after the plasmid was linearized with *Dra* I. A mixture of unlabelled and [α-³²P]GTP labelled CAT mRNA fragment (0.2 pmole in total) was mixed with the EGS^{CAT} RNA in amount of 4 pmole, 1 pmole, 1 pmole, 0.4 pmole and 0.2 pmole. Each mixture was incubated at 37°C in 50 mM Tris-Cl (pH 7.5), 100 mM NH₄Cl, and 25 mM MgCl₂ with enzyme for one hour. The reaction was stopped by addition of an equal volume of dye solution with excess EDTA and then subjected to a 5% polyacrylamide-7M urea gel. CAT mRNA alone was incubated without and with enzyme and loaded on the gel.

Primer extension analysis determining the precise site of EGS^{CAT}-directed cleavage by human RNAase P was conducted as follows. A reverse transcription reaction was performed on the uncleaved and cleaved CAT mRNA using an oligodeoxyribonucleotide 5'GGCCGTAATATCCAGCTGAACGG 3', complementary to nucleotide 129-107 of CAT mRNA. The reaction was incubated in 100 mM Tris.HCl (pH 8.3), 10 mM KCl, 6 mM MgCl₂, 10 mM DTT and 2 units AMV reverse transcriptase at 46°C for 2 hours. Labelled G, A, U, C were used as reference analyses of DNA sequences corresponding to the CAT mRNA template.

The precise site of cleavage of CAT mRNA was determined by primer extension analysis using an oligodeoxyribonucleotide primer complementary to nucleotide 129-107 of the RNA shows that the cleavage occurs between nucleotides 66 and 67, as expected.

The results of the EGS^{CAT} RNA directed cleavage of chloramphenicol acetyltransferase (CAT) mRNA were analyzed by gel electrophoresis. In the presence of EGS^{CAT} molecules, CAT mRNAs were cleaved to give rise to two products with the expected size. Analysis of the products of the reaction showed that the end groups contained 5' phosphoryl and 3' hydroxyl termini, the same as those normally generated by RNAase P. The results show conclusively that the specific cleavage of CAT mRNA is due to an EGS-directed RNAase P hydrolytic reaction.

For up to five-fold molar excess of EGS^{CAT} RNA to mRNA, the cleavage efficiency is proportional to the amount of EGS^{CAT} added. However, more than ten-fold excess of EGS^{CAT} molecules caused a decrease in the cleavage efficiency. One explanation for this is that EGS^{CAT} alone inhibits the enzymatic activity by competing with the mRNA-EGS complex for the enzyme. The reaction proceeds in a linear fashion for more than 3 hours at 37°C. Denaturation and reannealing of the oligonucleotides in the target complex did not improve the efficiency of cleavage. The reaction has an absolute requirement for Mg²⁺ with an optimal concentration of 25 mM, in contrast to that with tRNA^{Tyr} precursor as substrate, which has an optimal Mg²⁺ concentration of 2-10 mM.

### Example 3: Inhibition of Expression of CAT activity in green monkey CV-1 cells by EGS^{CAT}.

In order to test whether the EGS can function in *vivo,* the EGS^{CAT} sequence was inserted downstream of a mouse U6 snRNA gene promoter in a Bluescript™ vector. The EGS^{CAT} sequence can be transcribed by RNA polymerase III and the transcription can terminate at a T₅ cluster following the EGS sequence in either S100 extract or living cells. Green monkey fibroblast cells CV-1 were cotransfected with pCAT pEGS^{CAT} plasmids. After transfection with pCAT and pEGS^{CAT}, cells were harvested and CAT activity was assayed.

CV-1 cells were maintained in Dulbecco's modified Eagle medium that contained 10% fetal calf serum. One day prior to transfection cells were split 1:10 and plated in 60 mm Petri plates. Two hours prior to transfection cells were fed with 4.5 ml of fresh medium with 10% fetal calf serum. Transfection was performed using the calcium phosphate precipitation procedure using 2.5 µg of pCAT DNA and various amounts of pEGS^{CAT} DNA, ranging from one to 6.25 µg. Twenty-four hours after transfection, cells were harvested and cell extracts were assay for CAT activity.

The extract from cells cotransfected with EGS^{CAT} construct apparently decreased the conversion of chloramphenicol to its acetylated forms. The degree of inhibition was measured quantitatively by counting of the spots excised from a TLC plate. EGS^{CAT} cotransfection produced greater than 50% inhibition compared to the control with no EGS^{CAT} cotransfection. There was substantial loss of ability to inhibit CAT expression when a higher ratio of pEGS^{CAT} to pCAT was introduced. Similar experiments yielding approximately 70% of CAT activity have also been performed with human cells in tissue culture.

### Example 4: Preparation of Modified EGS enhancing degradation of target RNA by RNAase P.

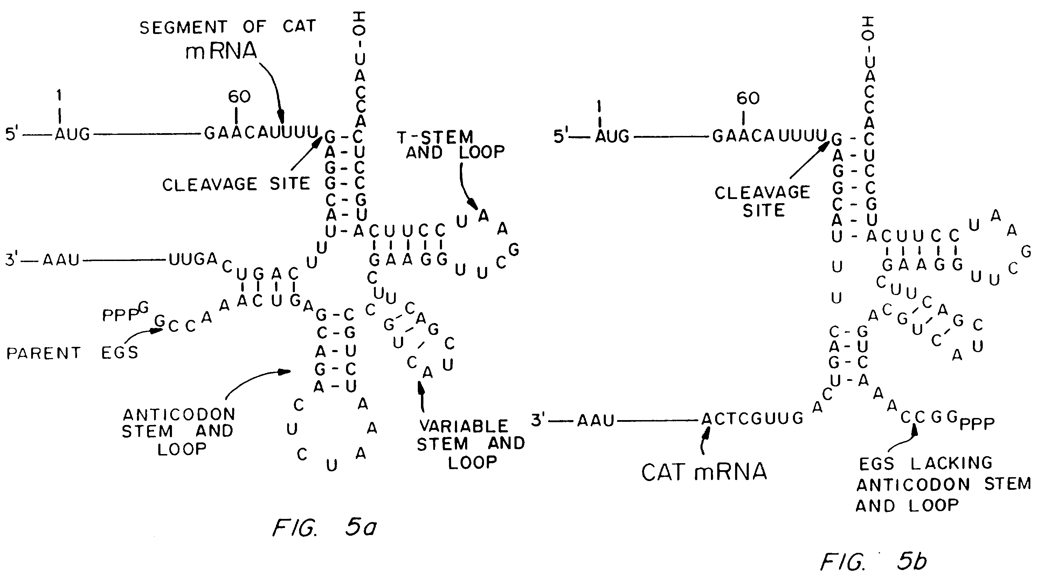

Two classes of EGS were designed, as shown in Figures 5 and 6. The first class involves deletions of large segments of the EGS as suggested in Example 1 and described in Examples 2 and 3. It has been found that the anticodon loop and stem can be deleted, as shown in Figure 5b (Sequence ID No. 4) and the EGS still promote cleavage of a target RNA by RNAase P. The variable loop and part of the variable loop can alternatively be deleted from the EGS and the EGS will promote cleavage with greater efficiency than the parent EGS molecule. The most efficient EGS was the one in which the anticodon stem and loop was deleted. This EGS promoted cleavage by human RNAase P of a target mRNA (CAT mRNA) at a rate 10-fold higher than the parent EGS. These deletions cannot be combined, however, to make an efficient EGS.

The second class of EGSs have changes in both the equivalent of the T loop, the variable loop, and the anticodon stem of the tRNA-like segment of the EGS. Three EGSs are shown in Figures 6a (Sequence ID No. 5), 6b (Sequence ID No. 6), and 6c (Sequence ID No. 7), respectively. Figure 6c shows the most efficient EGS, which directs cleavage by human RNAase P of a target RNA (CAT mRNA) in the appropriate complex at a rate approximately fifty to one hundred fold greater than the parent EGS.

These results apply to relative rates of cleavage at a particular site in a target mRNA. The absolute rates of cleavage at any particular site still depends on access of the EGS to that particular site.

### Example 5: Preparation of RNAs with randomized nucleotides.

Double stranded DNA templates were made by annealing of and enzymatically extending two overlapping synthetic oligonucleotides: TAATACGACTCACTATAGAACATTTTGAGGCATTTCAGTCAGTTGGCCAAACTG AGCAGAC (Sequence ID No. 8) and TGGTGAGGCATGAAGGNNNNGAACCTTCNNNNNGCAGATTTAGAGTCTGCTCAG TTTGGCC (Sequence ID No. 9), where the complementary sequences are underlined and the randomized nucleotides N were introduced during their machine synthesis by incorporating equimolar quantities of four nucleotides. These sequences create a chimeric tRNA gene that contains sequences from CAT mRNA and tRNA^{Tyr} from *E.coli.* A promoter for T7 bacteriophage RNA polymerase is included in Sequence ID No. 8. The extension was carried out with AMV reverse transcriptase at 46°C for two hours. Variant RNA pools were prepared by transcription with T7 polymerase in 40 mM Tris.Cl, pH 7.9, 6 mM MgCl₂ 10 mM dithiothreitol 2 mM spermidine 1 mM NTPs containing 20 µCi [³²-α] GTP at 37°C.

**The selection procedure.** RNA substrates were subjected to digestion with 30 units (one unit is defined as the amount of human RNAase P that will digest 1 pmol of tRNA precursor in 30 min at 37°C) for 2 hrs in the first three rounds of the selection procedure. For subsequent rounds of selection, the amount of the enzyme was reduced and incubation time was shortened so that less than 20% of substrate was cleaved. Cleaved products were separated from uncleaved substrates by electrophoresis on a 8% polyacrylamide/7 M urea gel. The purified product RNAs were reverse transcribed and amplified by PCR with Sequence ID No. 8 and TGGTGAGGCATGAAGG (Sequence ID No. 10) as primers using the Perkin Elmer RNA PCR kit. After each round of selective amplification, random mutations were introduced by performing the PCR with an error rate of 0.1% per each nucleotide position. PCR-generated double stranded DNAs regained the T7 promoter and leading sequence from primer Sequence ID No. 8, and were used to transcribe RNA for the next round of selection.

**Characterization of the selected RNAs and EGS RNA derived from them.** After eight cycles of selection, the resulting double-stranded DNAs were cloned into the Bluescript vector (Promega). Eighteen plasmid DNAs were sequenced using Sequenase 2.0 (U.S. Biochemicals, Cleveland, OH).

In order to test the abilities of EGSs derived from the individual variants selected above for CAT mRNA targeting, sequences corresponding to the EGS segment of each chimeric tRNA were amplified by PCR using primers Sequence ID No. 8 and AATACGACTCACTATAGGCCAACTGAGCAGAC (Sequence ID No. 11), which contains a promoter sequence for T7 polymerase, and RNAs were transcribed with T7 RNA polymerase. EGS-directed CAT mRNA cleavage was assayed in 10 µl of 50 mM Tris.Cl, pH 7.5, 10 mM MgCl₂ 100 mM NH₄Cl containing 0.25 pmol (1000 cpm) of substrate RNA and 1 or 5 pmol of EGS RNAs. Reaction mixtures were incubated at 37°C for 30 min with 10 units of RNase P from HeLa cells, followed by electrophoresis in 5% polyacrylamide/7 M urea gels.

The gels show a species of RNA migrating in the position expected for cleavage of substrate RNA in those lanes where the newly selected EGSs have been included in the reaction mixtures.

This method can be utilized to produce efficient EGSs for any RNAase P by randomizing other parts of the parent EGS sequence to select for EGSs with sequences other than those described herein that may be even more efficient.

### Summary of data in examples.

Although messenger RNAs are not natural substrates of RNAase P, complexes of mRNA-EGS have a common feature with precursors to tRNA in secondary or tertiary structure that forms the basis for the specificity of RNA cleavage by RNAase P. Given the secondary structures associated with RNA molecules, the choice of target site may play a crucial role in determining whether the substrate is accessible to EGS binding. Some target regions may participate in a high order structure which prevents the mRNA from binding EGS. Since high order structures can hinder the accessibility of certain sequences in a potential target to an EGS, some general methods, such as sensitivity to nucleases specific for single-stranded regions and analysis of sequences for regions required to be accessible to components of the translation apparatus, may be necessary to scan for suitable target sites in mRNA.

The method established in this work enables one to direct human RNAase P to cleave any target RNA *in vivo* with an appropriately designed proper external guide sequence (EGS). The method may provide a powerful new tool to block the function of specific RNAs. Theoretically, specific degradation of certain RNAs by RNAase P should be more powerful than the antisense RNA technique because RNAase P destroys the targeted RNA irreversibly and RNAase P, as well as the EGS, act catalytically since they can recycle many times, Li, et al., Proc. Natl. Acad. Sci. USA 89, 3185-3189 (1992) and Perreault and Altman, J. Mol. Biol. 226, 399-409 (1992).

Modifications and variations of the method and compositions to target any RNA for cleavage by eukaryotic RNAase P will be obvious to those skilled in the art from the foregoing detailed description. Such modifications and variations are intended to come within the scope of the appended claims.

## Claims

1. A recombinant ongonucleotide external guide sequence for targeting an RNA substrate for cleavage by eukaryotic RNase P comprising in order
nucleotide sequence including at least seven nucleotides complementary to and base pairing with the substrate immediately 3' to a site in the substrate to be cleaved to form a structure similar to an aminoacyl acceptor stem,
nucleotide sequence base pairing with itself to form a structure similar to the T stem and loop of a precursor tRNA,
nucleotide sequence forming structure similar to all or portions of the variable stem, the variable loop, the anticodon stem and the anticodon loop of a precursor tRNA, and nucleotide sequence including at least four nucleotides complementary to and base pairing with the substrate to form a structure similar to a dihydrouracil stem.

2. The guide sequence of Claim 1 wherein the external guide sequence comprises sequence forming a structure similar to the anticodon stem and loop or portions thereof but not the variable loop and stem of a precursor tRNA; or
comprises sequence forming a structure similar to the variable stem and loop or portions thereof but not the anticodon stem and loop of a precursor tRNA.

3. The guide sequence of Claim 1 or 2 wherein the external guide sequence is derived from a naturally occurring tRNA sequence modified in a region of the nucleotide sequence forming a structure similar to a structure selected from the group consisting of the T loop, T stem, anticodon stem, anticodon loop, variable stem and loop, to include one or more nucleotide bases not present in the naturally occurring tRNA sequence.

4. The guide sequence of any of Claims 1 to 3 wherein RNase P is targeted by the external guide sequence to cleave sequences selected from the group consisting of oncogenes, tumor suppressor genes, viral genes, and cellular mRNAs which encode proteins selected from the group consisting of enzymes, hormones, cofactors, antibodies, and growth factors and the external guide sequence includes regions of nucleotides that base pair with the sequence to be cleaved.

5. The guide sequence of any of Claims 1 to 4 in combination with yeast or mammalian RNase P.

6. The guide sequence of any of Claims 1 to 5 in combination with a pharmaceutical carrier selected from the group consisting of carriers suitable for topical, subcutaneous, parenteral, and enteral administration.

7. The guide sequence of any of Claims 1 to 6 and other sequence together forming a vector for introducing the external guide sequence into a cell containing the RNA targeted for cleavage.

8. The guide sequence of Claim 7 wherein the vector comprises retroviral sequence.

9. A method for specifically cleaving RNA comprising exposing *in vitro* the RNA to be cleaved to RNase P and a recombinant oligonucleotide external guide sequence as claimed in any of Claims 1 to 6.

10. The method of Claim 9 wherein the targeted RNA is intracellular and the RNase P is endogenous to the cell.

11. The method of Claim 9 or 10 further comprising providing an RNase P functional equivalent with the external guide sequence.

12. A method for selecting a population of external guide sequences for cleavage of a target RNA by RNase P with increased efficiency over a starting external guide sequence comprising
randomizing a section of the starting external guide sequence;
selecting for a subpopulation of the randomized sequences for their ability to be cleaved efficiently by RNase P;
amplifying those sequences cleaving more efficiently than the starting external guide sequence; and
repeating the selection and amplification steps; wherein the starting external guide sequences comprise a recombinant oligonucleotide external guide sequence as claimed in any of Claims 1 to 4.

13. A recombinant oligonucleotide external guide sequence as claimed in any of Claims 1 to 8 for use in medicine.

14. A molecule comprising an external guide sequence according to any of Claims 1 to 8 and an RNA subunit of RNase P.

## Patentansprüche

1. Externe rekombinante Oligonukleotidführungssequenz zum Erkennen eines RNA-Substrats zur Spaltung durch eukaryontische Rnase P, die in dieser Reihenfolge die folgenden Nukleotidsequenzen umfaßt:
eine Nukleotidsequenz, die mindestens sieben mit dem Substrat unmittelbar 3' zu einer zu spaltenden Stelle im Substrat komplementäre und damit eine Basenpaarung eingehende Nukleotide umfaßt, wobei eine einem Aminoacylakzeptorstamm ähnliche Struktur gebildet wird,
eine Nukleotidsequenz, die mit sich selbst eine Basenpaarung eingeht, wobei eine dem T-Stamm und der Schleife einer Vorläufer-tRNA ähnliche Struktur gebildet wird,
eine Nukleotidsequenz, die eine der Gesamtheit aus dem variablen Stamm, der variablen Schleife, dem Anticodonstamm und der Anticodonschleife einer Vorläufer-tRNA oder Teilen hiervon ähnliche Struktur bildet, und
eine Nukleotidsequenz, die mindestens vier mit dem Substrat komplementäre und damit eine Basenpaarung eingehende Nukleotide umfaßt, wobei eine einem Dihydrourazilstamm ähnliche Struktur gebildet wird.

2. Führungssequenz nach Anspruch 1, wobei die externe Führungssequenz eine Sequenz, die eine dem Anticodonstamm und der Schleife oder Teilen hiervon, jedoch nicht der variablen Schleife und dem variablen Stamm einer Vorläufer-tRNA ähnliche Struktur bildet, oder eine Sequenz umfaßt, die eine dem variablen Stamm und der variablen Schleife oder Teilen hiervon, jedoch nicht dem Anticodonstamm und der Schleife einer Vorläufer-tRNA ähnliche Struktur bildet.

3. Führungssequenz nach Anspruch 1 oder 2, wobei die externe Führungssequenz von einer natürlich vorkommenden, in einem Bereich der Nukleotidsequenz modifizierten tRNA-Sequenz herrührt, die eine zu einer unter T-Schleifen, T-Stämmen, Anticodonstämmen, Anticodonschleifen, variablen Stämmen und variablen Schleifen ausgewählten Struktur ähnliche Struktur bildet, wobei eine oder mehrere in der natürlich vorkommenden tRNA-Sequenz nicht vorhandene Nukleotidbasen enthalten sind.

4. Führungssequenz nach einem der Ansprüche 1 bis 3, wobei die RNase P durch die externe Führungssequenz so erkannt wird, daß Sequenzen gespalten werden, die unter Oncogenen, Tumorsuppressorgenen, Virusgenen und zellulären mRNAs mit Kodierung für unter Enzymen, Hormonen, Cofaktoren, Antikörpern und Wachstumsfaktoren ausgewählten Proteinen ausgewählt sind, und die externe Führungssequenz Bereiche von Nukleotiden umfaßt, die mit der zu spaltenden Sequenz eine Basenpaarung eingehen.

5. Führungssequenz nach einem der Ansprüche 1 bis 4 in Kombination mit Hefe- oder Säugetier-RNase P.

6. Führungssequenz nach einem der Ansprüche 1 bis 5 in Kombination mit einem unter Trägern mit Eignung zur topischen, subkutanen, parenteralen und enteralen Verabreichung ausgewählten Träger.

7. Führungssequenz nach einem der Ansprüche 1 bis 6 und eine andere Sequenz, die zusammen einen Vektor zur Einführung der externen Führungssequenz in eine Zelle, die die als zu spaltende RNA erkannte RNA enthält, bilden.

8. Führungssequenz nach Anspruch 7, wobei der Vektor eine retrovirale Sequenz umfaßt.

9. Verfahren zur spezifischen Spaltung von RNA durch in vitro-Einwirkenlassen von RNase P und einer externen rekombinanten Oligonukleotidführungssequenz nach einem der Ansprüche 1 bis 8 auf die zu spaltende RNA.

10. Verfahren nach Anspruch 9, wobei die erkannte RNA intrazellulär und die RNase P endogen bezüglich der Zelle sind.

11. Verfahren nach Anspruch 9 oder 10, das des weiteren ein Bereitstellen einer mit der externen Führungssequenz funktionell äquivalenten Rnase P umfaßt.

12. Verfahren zur Auswahl einer Population externer Führungssequenzen zur Spaltung einer Target-RNA durch RNase P mit erhöhter Effizienz gegenüber einer externen Ausgangsführungssequenz durch
Randomisieren eines Bereichs der externen Ausgangsführungssequenz;
Auswählen bezüglich einer Subpopulation der randomisierten Sequenzen hinsichtlich ihrer Fähigkeit, durch RNase P wirksam gespalten zu werden;
Amplifizieren derjenigen Sequenzen, die effizienter spalten als die externen Ausgangsführungssequenzen, und
Wiederholen der Auswahl und Amplifikation, wobei die externen Ausgangsführungssequenzen eine externe rekombinante Oligonukleotidführungssequenz nach einem der Ansprüche 1 bis 4 umfassen.

13. Externe rekombinante Oligonukleotidführungssequenz nach einem der Ansprüche 1 bis 8 zur Verwendung in der Medizin.

14. Molekül, das eine externe Führungssequenz nach einem der Ansprüche 1 bis 8 und eine RNA-Untereinheit von Rnase P umfaßt.

## Revendications

1. Séquence de guidage externe d'oligonucléotides recombinants destinée à cibler un substrat ARN qui doit être clivé par une RNase P d'eucaryote comprenant dans l'ordre
une séquence nucléotidique incluant au moins sept nucléotides complémentaires et capables de former des appariements de bases avec le substrat immédiatement en 3' jusqu'à un site dans le substrat qui doit être clivé pour former une structure similaire à une tige "accepteur" d'aminoacyles,
une séquence nucléotidique capable de former des appariements de bases avec elle-même pour former une structure similaire à la tige et à la boucle T d'un ARNt précurseur,
une séquence nucléotidique formant une structure similaire à la totalité ou à des parties de la tige variable, la boucle variable, la tige anticodon et la boucle anticodon d'un ARNt précurseur, et
une séquence nucléotidique incluant au moins quatre nucléotides complémentaires et capables de former des appariements de bases avec le substrat pour former une structure similaire à une tige dihydro-uracile.

2. Séquence de guidage selon la revendication 1, dans laquelle la séquence de guidage externe comprend une séquence formant une structure similaire à la tige et à la boucle anticodon ou à des parties de celles-ci mais pas à la boucle et à la tige variables d'un ARNt précurseur ; ou
qui comprend une séquence formant une structure similaire à la tige et à la boucle variables ou à des parties de celles-ci mais pas à la tige et à la boucle anticodon d'un ARNt précurseur.

3. Séquence de guidage selon la revendication 1 ou 2, dans laquelle la séquence de guidage externe est dérivée d'une séquence d'ARNt naturelle modifiée dans une région de la séquence nucléotidique formant une structure similaire à une structure choisie dans le groupe constitué par la boucle T, la tige T, la tige anticodon, la boucle anticodon, la tige et la boucle variables, de façon à inclure une ou plusieurs bases nucléotidiques qui ne sont pas présentes dans la séquence d'ARNt naturelle.

4. Séquence de guidage selon l'une quelconque des revendications 1 à 3, dans laquelle la RNase P est ciblée par la séquence de guidage externe pour cliver des séquences choisies dans le groupe constitué par les oncogènes, les gènes suppresseurs de tumeurs, les gènes viraux, et les ARNm cellulaires qui codent pour des protéines choisies dans le groupe constitué par les enzymes, les hormones, les cofacteurs, les anticorps, et les facteurs de croissance et dans laquelle la séquence de guidage externe inclut des régions de nucléotides dont les bases s'apparient à celles de la séquence à cliver.

5. Séquence de guidage selon l'une quelconque des revendications 1 à 4 combinée à une RNase P de levure ou de mammifère.

6. Séquence de guidage selon l'une quelconque des revendications 1 à 5 combinée à un véhicule pharmaceutique choisi dans le groupe constitué par des véhicules appropriés pour une administration par voie locale, sous-cutanée, parentérale, et entérale.

7. Séquence de guidage selon l'une quelconque des revendications 1 à 6 et autre séquence formant ensemble un vecteur destiné à introduire la séquence de guidage externe dans une cellule contenant l'ARN ciblé pour le clivage.

8. Séquence de guidage selon la revendication 7, dans laquelle le vecteur comprend une séquence rétrovirale.

9. Procédé de clivage spécifique d'ARN consistant à exposer *in vitro* l'ARN qui doit être clivé à la RNase P et à une séquence de guidage externe d'oligonucléotides recombinants selon l'une quelconque des revendications 1 à 6.

10. Procédé selon la revendication 4, dans lequel l'ARN ciblé est intracellulaire et la RNase P est endogène à la cellule.

11. Procédé selon la revendication 9 ou 10 consistant en plus à fournir une RNase P équivalente du point de vue des fonctions à la séquence de guidage externe.

12. Procédé de sélection d'une population de séquences de guidage externes destinées à cliver un ARN cible par le moyen d'une RNase P avec une efficacité accrue par rapport à une séquence de guidage externe de départ consistant à :
répartir au hasard un fragment de la séquence de guidage externe de départ ;
opérer une sélection pour obtenir une sous-population de séquences réparties au hasard en fonction de leur capacité à être clivées efficacement par une RNase P ;
amplifier les séquences clivant plus efficacement que la séquence de guidage externe de départ ; et
répéter les étapes de sélection et d'amplification ; dans lequel les séquences de guidage externes de départ comprennent une séquence de guidage externe d'oligonucléotides recombinants selon l'une quelconque des revendications 1 à 4.

13. Séquence de guidage externe d'oligonucléotides recombinants selon l'une quelconque des revendications 1 à 8 pour une utilisation médicale.

14. Molécule comprenant une séquence de guidage externe selon l'une quelconque des revendications 1 à 8 et une sous-unité d'ARN de RNase P.
